# EUROPEAN PATENT APPLICATION

(11) **EP 4 180 005 A1**
(43) Date of publication of application: **17.05.2023**
(21) Application number: 21837791.9
(22) Date of filing: 07.07.2021
(51) Int. Cl.: A61F 2/07

(54) **STENT GRAFT**

(30) Priority: 07.07.2020 JP 2020116955
(71) Applicant: Hiroshima University, Higashi-Hiroshima-shi Hiroshima 739-8511 (JP); Goodman Co., Ltd., Aichi 460-0008 (JP)
(72) Inventor: TAKAHASHI, Shinya, Hiroshima-shi, Hiroshima 739-8511 (JP); KATAYAMA, Keijiro, Hiroshima-shi, Hiroshima 739-8511 (JP); SUEDA, Taijiro, Hiroshima-shi, Hiroshima 739-8511 (JP); HIROTA, Etsuko, Seto-shi, Aichi 489-0976 (JP); ASAI, Motoki, Seto-shi, Aichi 489-0976 (JP)
(74) Representative: Epping - Hermann - Fischer
(86) International application number: PCT/JP2021/025625
(87) International publication number: WO 2022/009923

(57) **Abstract**

A stent graft (1A) includes a tubular member (2A), a plurality of stent rings (2B), and an uncovered section (7). The tubular member (2A) extends between a proximal end (21) and a distal end (22). The plurality of stent rings (2B) extend while alternatingly bending toward the proximal end (21) side and toward the distal end (22) side. The plurality of stent rings include a plurality of fluctuating rings (5) and a small fixed ring (3). The plurality of fluctuating rings have a first region in which the amplitude is a first value and a second region in which the amplitude is a second value smaller than the first value. The small fixed ring (3) exhibits a maximum amplitude which is a third value which is smaller than the first value. The uncovered section (7) extends while alternatingly bending toward a base end side and toward a tip end side.

## Description

### Technical Field

The present invention relates to a stent graft.

### Background Art

Stent-graft insertion is known as a medical treatment for an aortic aneurysm. With stent-graft insertion, a catheter fit with a deflated stent graft is inserted into the aorta and moved to the affected area, after which the stent graft is placed in position. The stent graft that has been placed at the affected area then expands from the restoring force of the stent, and sticks to the inner wall of the blood vessel. As a result, the stent graft inhibits the flow of blood into the aortic aneurysm. Because stent-graft insertion does not require surgery where a large incision would be made in the body, it is a minimally invasive medical treatment.

Patent Literature 1 discloses a stent graft to be placed in an aortic arch. This stent graft has, as a structure for accommodating the sharp bend of the aortic arch, a graded structure. The graded structure is formed by changing, in the circumferential direction, the length in the longitudinal direction between the mountains and valleys of linear struts that form the tubular unit of the stent graft.

### Citation List

### Patent Literature

Patent Literature 1: WO-A1-2017/047569

### Summary of Invention

The end portion of the stent graft where an opening is formed must very closely contact the inner wall of the blood vessel. With respect to this, with the stent graft described in Patent Literature 1, there are cases in which the closeness of contact of the end portion with respect to the inner wall of the blood vessel decreases.

The object of the present invention is to provide a stent graft in which the end portion where the opening is formed can be made to suitably closely contact the inner wall of a blood vessel.

A stent graft for the present invention includes a tubular member, a plurality of stent rings, and an uncovered section. The tubular member extends between a proximal end that is an end portion on one side in a predetermined axial direction, and a distal end that is an end portion on the other side in the axial direction. The plurality of stent rings are fixed to a side wall of the tubular member and formed of struts extending in a ring shape in a circumferential direction of the tubular member, while alternatingly bending toward the proximal end side and toward the distal end side. The plurality of stent rings include at least a fluctuating ring and a small fixed ring. The fluctuating ring includes a first region in which an amplitude in the axial direction is a first value, and a second region in which an amplitude is a second value that is smaller than the first value. A maximum value of an amplitude of the small fixed ring is a third value that is smaller than the first value. The uncovered section is formed of a strut, at least a portion of which is disposed on a side opposite the distal end side with respect to the proximal end. The strut is extending in a ring shape while alternatingly bending toward a base end side that is an end portion on the distal end side, and toward a tip end side that is an end portion on the side opposite the distal end side.

With the stent graft, the portion of the fluctuating rings of the tubular member can be made to closely contact the inner wall of the sharply curved portion of the blood vessel. Also, with the stent graft, the uncovered section can be made to closely contact the inner wall of the blood vessel. Also, with the stent graft, the proximal end of the tubular member where the opening is formed can be made to suitably closely contact the inner wall of the blood vessel by optimizing the angle of the proximal end of the uncovered section.

In the present invention, the small fixed ring may be adjacent on the distal end side with respect to the uncovered section, and the fluctuating ring may be adjacent on the distal end side with respect to the small fixed ring. In this case, with the stent graft, the proximal end of the tubular member where the opening is formed can be made to more suitably closely contact the inner wall of the blood vessel by the uncovered section and the small fixed ring.

In the present invention, the base end of the uncovered section may be positioned on the distal end side with respect to the end portion on the proximal end side of the small fixed ring, and the tip end of the uncovered section may be positioned on the side opposite the distal end side with respect to the end portion on the proximal end side of the small fixed ring. The fluctuating ring may be adjacent on the distal end side with respect to the small fixed ring. With the stent graft, the proximal end of the tubular member where the opening is formed can be made to more suitably closely contact the inner wall of the blood vessel by the uncovered section and the small fixed ring.

In the present invention, positions in the circumferential direction of a plurality of poles of the base end of the uncovered section and a plurality of poles on the proximal end side of the small fixed ring may be different. The stent graft can inhibit interference between the uncovered section and the small fixed ring.

In the present invention, positions in the circumferential direction of a plurality of poles of the base end of the uncovered section and a plurality of poles on the proximal end side of the small fixed ring may be the same. With the stent graft, the tubular member can be more effectively expanded outward at the overlapping portion of the uncovered section and the small fixed ring to closely contact the blood vessel.

In the present invention, the uncovered section may be such that an extending direction from the base end toward the tip end extends in a direction away from a center line passing through a center of the tubular member and extending in the axial direction. With the stent graft, the uncovered section can be made to closely contact the inner wall of the blood vessel by elastic force generated in the uncovered section.

In the present invention, the uncovered section may include a first portion and a second portion. The first portion where an angle of the extending direction with respect to a reference direction may be a first angle. The reference direction may extend on the side opposite the distal end side with respect to the proximal end and is parallel to the center line. The second portion may include the tip end and where an angle of the extending direction with respect to the reference direction is smaller than the first angle. The stent graft can reduce the possibility of the tip end of the uncovered section damaging the inner wall of the blood vessel when the second portion of the uncovered section is brought into contact with the inner wall of the blood vessel.

In the present invention, the plurality of stent rings may further include a large fixed ring in which a maximum value of an amplitude is a fourth value that is larger than the third value, and which is disposed on the distal end side of the fluctuating ring and the small fixed ring. With the stent graft, the large fixed ring can be made to closely contact the portion extending linearly from the sharply curved portion of the blood vessel.

In the present invention, the uncovered section may be such that the base end is fixed to an inner surface of the proximal end of the tubular member. With the stent graft, the proximal end of the tubular member can be made to closely contact the blood vessel.

In the present invention, the plurality of stent rings may be fixed to an outer surface of the tubular member. The stent graph can reduce the possibility of a variety of devices (for example, a guide wire) that pass through the inside of the tubular member catching on the plurality of stent rings.

In the present invention, the plurality of stent rings may be fixed to an inner surface of the tubular member. With the stent graft, the tubular member can be expanded outward by the elastic force of the plurality of stent rings to closely contact the inner wall of the blood vessel.

In the present invention, the third value may be smaller than the second value. The stent graft can further increase the force that causes the proximal end of the tubular member where the opening is formed to suitably closely contact the inner wall of the blood vessel.

In the present invention, there may be a plurality of the fluctuating rings, and the single small fixed ring. The stent graft can further increase the closeness of contact with the inner wall of the sharply curved portion of the blood vessel.

In the present invention, the plurality of poles on the proximal end side of the fluctuating ring may be arranged on a virtual line extending in a direction orthogonal to the axial direction along the circumferential direction. The stent graft can increase the force that causes the proximal end of the tubular member where the opening is formed to suitably closely contact the inner wall of the blood vessel.

### Brief Description of the Drawings

Fig. 1 is a side view of a stent graft 1A.
Fig. 2 is an enlarged side view of the area near a proximal end 21 of the stent graft 1A.
Fig. 3 is an developed view of a small fixed ring 3.
Fig. 4 is an enlarged side view of the area near a first fluctuating ring 51 to a third fluctuating ring 53 of the stent graft 1A.
Fig. 5 is an developed view of the first fluctuating ring 51.
Fig. 6 is an developed view of an nth fluctuating ring 5n.
Fig. 7 is an enlarged side view of the area near a distal end 22 of the stent graft 1A.
Fig. 8 is an developed view of an mth large fixed ring 6m.
Fig. 9 is a side view of the stent graft 1A.
Fig. 10 is an developed view of an uncovered section 7 and the small fixed ring 3.
Fig. 11 is an enlarged side view of the area near the proximal end 21 of the stent graft 1A.
Fig. 12 is a view showing the stent graft 1A placed in a thoracic aorta 8.
Fig. 13 is an enlarged view of a portion of Fig. 12.
Fig. 14 is an enlarged side view in frame format of the uncovered section 7 and the small fixed ring 3 in a modified example.

### Description of Embodiments

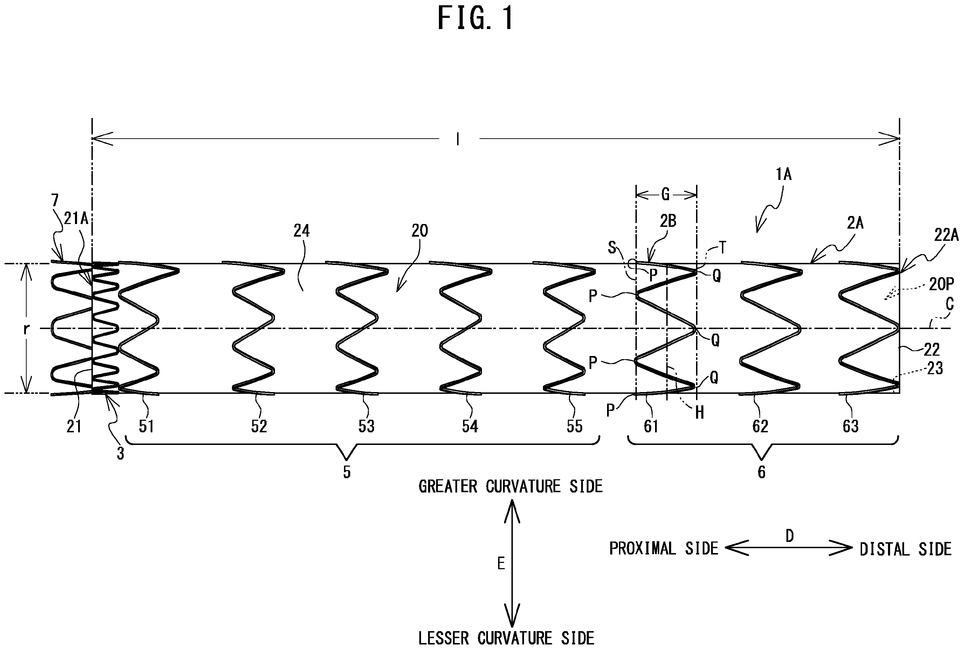

A stent graft 1A of one embodiment of the present invention will be described. In the present embodiment, unless otherwise specified, direction and distance and the like will be described assuming that the stent graft 1A is in a linearly extended state. A left side and a right side in Fig. 1 define a proximal side and a distal side, respectively, of the stent graft 1A. A direction extending between the proximal side and the distal side will be defined as an axial direction D. An upper side and a lower side in Fig. 1 define a greater curvature side and a lesser curvature side, respectively, of the stent graft 1A. A direction extending between the greater curvature side and the lesser curvature side will be defined as an expansion-contraction direction E.

### Overview of stent graft 1A

The stent graft 1A is used to treat, by stent-graft insertion, an aortic aneurysm formed near an aortic arch of a thoracic aorta, or an aortic dissection in which a proximal dissection cavity (entry) has formed near the aortic arch. As shown in Fig. 1, the stent graft 1A has, as the main constituent elements, a tubular member 2A, a plurality of stent rings 2B, and an uncovered section 7.

The stent graft 1Ais fitted into a delivery system, not shown in the drawings, in a state folded up inside. The delivery system is inserted into the body through an incision opened in a blood vessel at the base of a leg, and guided to the affected area. Then, the stent graft 1A is placed at the affected area. The tubular member 2A of the stent graft 1A is expanded by the elastic force of the plurality of stent rings 2B to closely contact the inside of the affected area. The tubular member 2A inhibits blood from flowing into a portion of a blood vessel where there is an aneurysm or dissection cavity, thereby protecting the blood vessel. By optimizing the angle of the proximal end, the uncovered section 7 of the stent graft 1A inhibits the occurrence of a phenomenon (end leak) where blood leaks from an opening 21A of the tubular member 2A placed at the affected area and flows into the aortic aneurysm or dissection cavity. Note that hereinafter, unless otherwise specified, each structure of the stent graft 1A will be described assuming that the tubular member 2A is in an expanded state (the state shown in Fig. 1, etc.) by the plurality of stent rings 2B.

### Tubular member 2A

The tubular member 2A is an artificial blood vessel (graft) with low water permeability. The tubular member 2A is formed by synthetic-resin graft material. The tubular member 2A has a hollow, long, thin structure, and extends along the axial direction D. Of both end portions in the axial direction D of the tubular member 2A, the end portion on the proximal side will be referred to as a proximal end 21, and the end portion on the distal side will be referred to as a distal end 22. The tubular member 2A extends between the proximal end 21 and the distal end 22. The cross-sectional shape when a side wall 20 of the tubular member 2A is cut by a plane orthogonal to the axial direction D is circular. An inner surface 23 of the side wall 20 has an internal cavity 20P that passes through in the axial direction D. The diameter r of the internal cavity 20P is constant between the proximal end 21 and the distal end 22. The stent graft 1A fits inside a delivery system and moves inside the blood vessel along a guide wire. A virtual line extending in the axial direction D and passing through the center of the internal cavity 20P of the tubular member 2A will be defined as a center line C. The direction following an outer surface 24 of the side wall 20 of the tubular member 2A and intersecting the axial direction D will be defined as a circumferential direction.

A thickness d of the tubular member 2A, a length l in the axial direction D from the proximal end 21 to the distal end 22 of the tubular member 2A, and the diameter r, are not particularly limited. As an example, the thickness d is equal to or less than 0.1 mm, the length l is 100 to 200 mm, and the diameter r is 20 to 45 mm. The diameter r need not be uniform from the proximal end 21 to the distal end 22 of the tubular member 2A. The cross-sectional shape when the side wall 20 of the tubular member 2A is cut by a plane orthogonal to the axial direction D need not be circular, and may be polygonal or ellipse, or the like.

### Plurality of stent rings 2B

The plurality of stent rings 2B are stent struts, each of which extends in a ring shape along the circumferential direction of the tubular member 2A. More specifically, each of the plurality of stent rings 2B is a wire having a cross-sectional shape that is circular or square with curved corners. Each of the plurality of stent rings 2B extends in a ring shape while alternatingly bending in the axial direction D toward the proximal side and toward the distal side. In other words, the plurality of stent rings 2B extend in a ring shape along the outer surface 24 of the side wall 20 of the tubular member 2A while alternatingly bending toward the proximal end 21 side and toward the distal end 22 side of the tubular member 2A. The plurality of stent rings 2B are fixed by being sewn on with sutures to the outer surface 24 of the side wall 20 of the tubular member 2A. Note that the method by which the plurality of stent rings 2B are fixed to the tubular member 2A may be another method. For example, the plurality of stent rings 2B may be fixed to the tubular member 2A by an adhesive or tape. Also, for example, the plurality of stent rings 2B may be disposed between a member equivalent to the tubular member 2A and the tubular member 2A, and fixed to the tubular member 2A by adhering the member equivalent to the tubular member 2A to the tubular member 2A with heat or the like.

The plurality of stent rings 2B include a small fixed ring 3, a plurality of fluctuating rings 5, and a plurality of large fixed rings 6, which will be described later. The thickness of the struts forming the small fixed ring 3, the plurality of fluctuating rings 5, and the plurality of large fixed rings 6 need not be the same. The small fixed ring 3, the plurality of fluctuating rings 5, and the plurality of large fixed rings 6 will be described in detail later.

Each of a plurality of poles where each of the plurality of stent rings 2B bends back from the proximal end 21 side to the distal end 22 will be referred to as a proximal pole P. In other words, the proximal pole P corresponds to a pole on the proximal end 21 side, of each of the plurality of stent rings 2B. The proximal pole P curves in a protruding shape toward the proximal end 21 side. A ring-shaped virtual line formed by connecting a plurality of line segments extending between two adjacent proximal poles P will be referred to as a proximal envelope curve S. Each of a plurality of poles where each of the plurality of stent rings 2B bends back from the distal end 22 side to the proximal end 21 side will be referred to as a distal pole Q. In other words, the distal pole Q corresponds to a pole on the distal end 22 side, of each of the plurality of stent rings 2B. The distal pole Q curves in a protruding shape toward the distal end 22 side. A ring-shaped virtual line formed by connecting a plurality of line segments extending between two adjacent distal poles Q will be referred to as a distal envelope curve T. The distance in the axial direction D between the proximal envelope curve S and the distal envelope curve T will be defined as an amplitude G.

The center position in the axial direction D between the proximal envelope curve S and the distal envelope curve T will be defined as a center position H. The center position H is disposed between the proximal envelope curve S and the distal envelope curve T in the axial direction D, and extends in a ring shape. The reciprocal of the number of the proximal poles P and the distal poles Q will be referred to as a bend cycle W.

### Small fixed ring 3

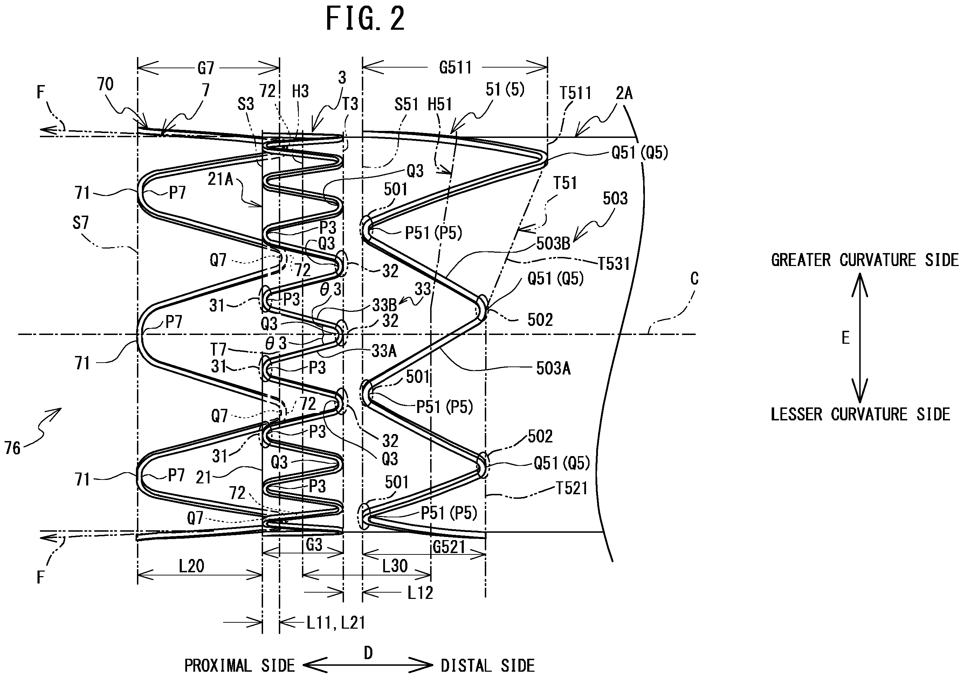

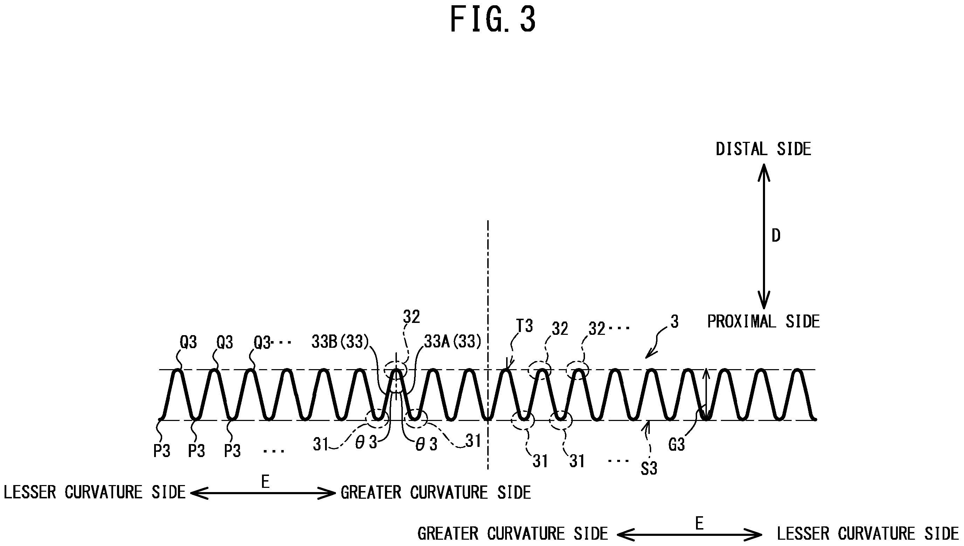

As shown in Fig. 2, the small fixed ring 3 is disposed such that the positions of the proximal end 21 of the tubular member 2A and a plurality of proximal poles P3 of the small fixed ring 3 are the same position in the axial direction D. That is, the plurality of proximal poles P3 of the small fixed ring 3 are disposed along the proximal end 21 of the tubular member 2A. Fig. 3 shows a state in which the small fixed ring 3 is cut at a portion corresponding to the end portion on the lesser curvature side in the expansion-contraction direction E, and laid out in a plane. The number of each of the plurality of proximal poles P3 and a plurality of distal poles Q3 of the small fixed ring 3 is, for example, 18, and a bend cycle W3 is, for example, 1/18.

As shown in Fig. 2 and Fig. 3, the small fixed ring 3 has curved portions 31 and 32, and a linear portion 33. The curved portion 31 is a portion near the each of the plurality of proximal poles P3 of the small fixed ring 3. The curved portion 32 is a portion near each of the plurality of distal poles Q3 of the small fixed ring 3. The linear portion 33 is a portion extending linearly between the curved portions 31 and 32 of the small fixed ring 3. A curvature R3 of the curved portions 31 and 32 is the same, and is 0.6 mm, for example. The linear portion 33 extending inclined on the lesser curvature side with respect to the axial direction D from the curved portion 32 toward the proximal side will be referred to as a linear portion 33A. The linear portion 33 extending inclined on the greater curvature side with respect to the axial direction D from the curved portion 32 toward the proximal side will be referred to as a linear portion 33B. The linear portions 33A and 33B are disposed symmetrically about a reference axis extending in the axial direction D through the curved portion 32 to which they each connect. An angle θ3 formed between the linear portion 33 and the axial direction D is, for example, 14.5°.

A proximal envelope curve S3 that connects the plurality of proximal poles P3, and a distal envelope curve T3 that connects the plurality of distal poles Q3 are each orthogonal to the axial direction D. The directions in which the proximal envelope curve S3 and the distal envelope curve T3 extend are parallel to each other. A center position H3 in the axial direction D of the proximal envelope curve S3 and the distal envelope curve T3 is orthogonal to the axial direction D. A third amplitude G3 which is the distance between the proximal envelope curve S3 and the distal envelope curve T3 is constant along the entire ring-shaped area of the small fixed ring 3, and is, for example, 6.5 mm.

### Plurality of fluctuating rings 5

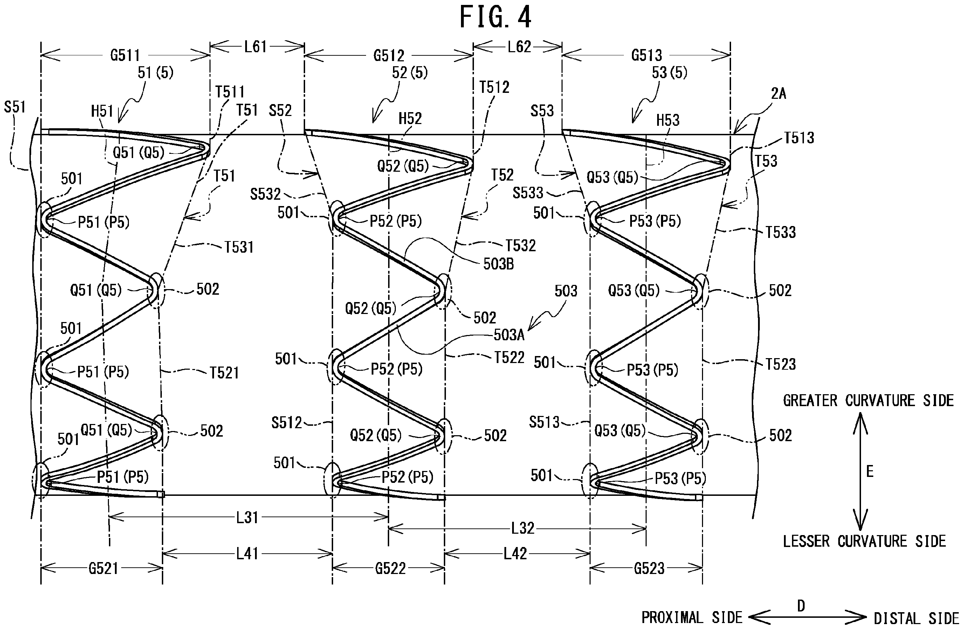

As shown in Fig. 1, the plurality of fluctuating rings 5 include a first fluctuating ring 51, a second fluctuating ring 52, a third fluctuating ring 53, a fourth fluctuating ring 54, and a fifth fluctuating ring 55. The first fluctuating ring 51 to the fifth fluctuating ring 55 are arranged in the order of the first fluctuating ring 51, the second fluctuating ring 52, the third fluctuating ring 53, the fourth fluctuating ring 54, and the fifth fluctuating ring 55, from the proximal end 21 side to the distal end 22 side of the tubular member 2A. As shown in Fig. 1 and Fig. 4, the shapes of the second fluctuating ring 52 to the fifth fluctuating ring 55 are the same. The shape of the first fluctuating ring 51 is different from the shape of the second fluctuating ring 52 to fifth fluctuating ring 55. This will be described in more detail later.

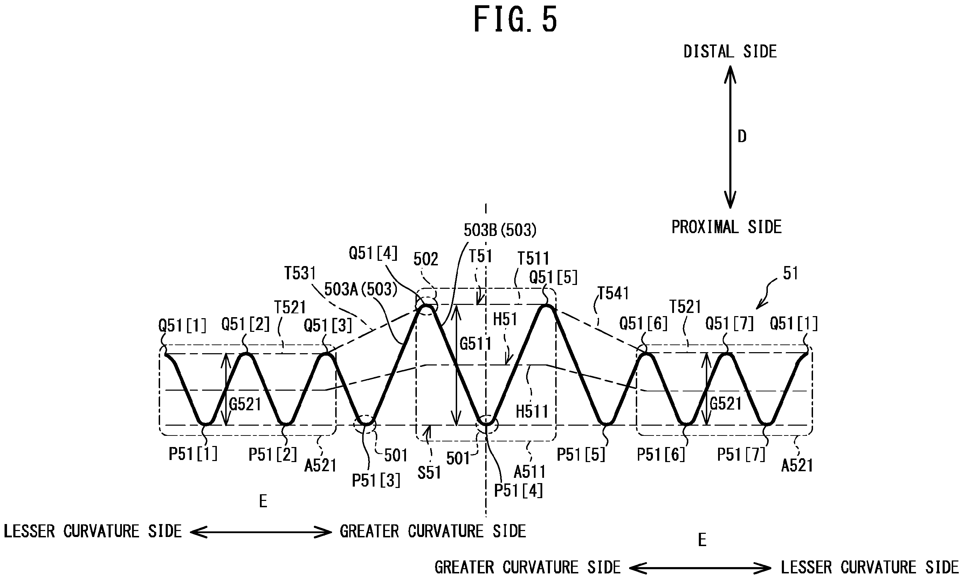

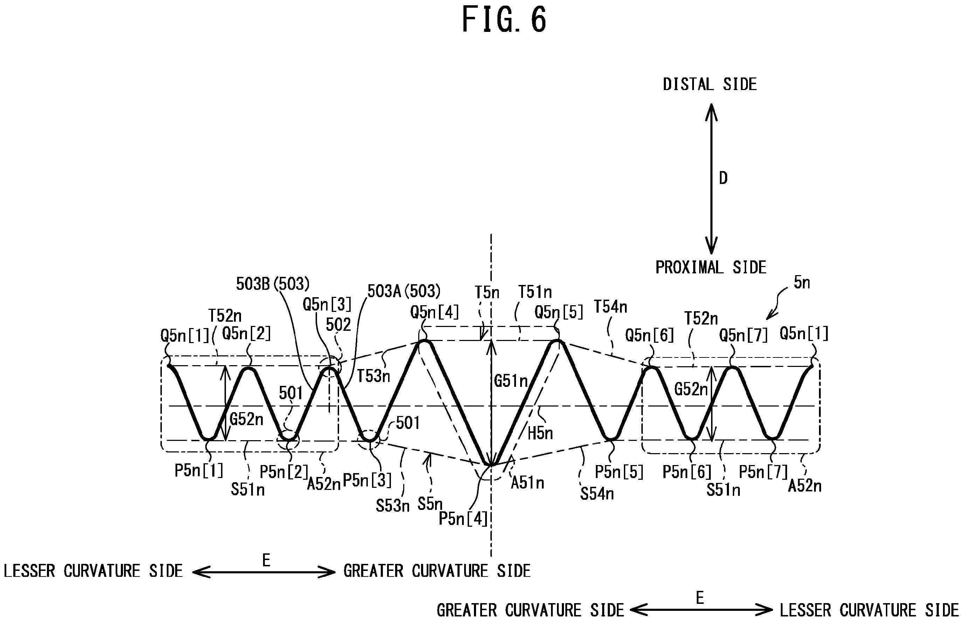

Fig. 5 shows a state in which the first fluctuating ring 51 is cut at a portion corresponding to the end portion on the lesser curvature side in the expansion-contraction direction E, and laid out in a plane. Fig. 6 shows a state in which each of the second fluctuating ring 52 to the fifth fluctuating ring 55 is cut at a portion corresponding to the end portion on the lesser curvature side in the expansion-contraction direction E, and laid out in a plane.

As shown in Fig. 2, Fig. 4, Fig. 5, and Fig. 6, the number of each of a plurality of proximal poles P5 (refer to Fig. 2 and Fig. 4) and a plurality of distal poles Q5 (refer to Fig. 2 and Fig. 4) of each of the plurality of fluctuating rings 5 is the same with the first fluctuating ring 51 to the fifth fluctuating ring 55, and is, for example, 7. A bend cycle W5 is, for example, 1/7. Each of the plurality of fluctuating rings 5 has curved portions 501 and 502 and a linear portion 503. The curved portion 501 is a portion near each of the plurality of proximal poles P5. The curved portion 502 is a portion near each of the plurality of distal poles Q5. The linear portion 503 is a portion extending linearly between the curved portions 501 and 502. The linear portion 503 extending inclined on the lesser curvature side with respect to the axial direction D from the curved portion 502 toward the proximal side will be referred to as a linear portion 503A. The linear portion 503 extending inclined on the greater curvature side with respect to the axial direction D from the curved portion 502 toward the proximal side will be referred to as a linear portion 503B. The linear portions 503A and 503B are disposed symmetrically about a reference axis extending in the axial direction D through the curved portion 502 to which they each connect.

Hereinafter, each of the first fluctuating ring 51 to the fifth fluctuating ring 55 may be written as the Nth (with N being any integer from 1 to 5) fluctuating ring 5N. Each of the second fluctuating ring 52 to the fifth fluctuating ring 55 may be written as the nth (with n being any integer from 2 to 5) fluctuating ring 5n.

### First fluctuating ring 51

As shown in Fig. 2, the first fluctuating ring 51 is disposed adjacent on the distal end 22 (refer to Fig. 1) side with respect to the small fixed ring 3. A proximal envelope curve S51 that connects a plurality of proximal poles P51 of the first fluctuating ring 51 extends in the circumferential direction and is orthogonal to the axial direction D. In other words, the plurality of proximal poles P51 of the first fluctuating ring 51 are lined up on a virtual line (proximal envelope curve S51) extending in the circumferential direction along the outer surface 24 of the tubular member 2A and orthogonal to the axial direction D. The positions in the axial direction D of the plurality of proximal poles P51 of the first fluctuating ring 51 are the same.

The distal envelope curve T3 of the small fixed ring 3 and the proximal envelope curve S51 of the first fluctuating ring 51 are parallel to each other. A distance L12 in the axial direction D between the distal envelope curve T3 and the proximal envelope curve S51 corresponds to the distance in the axial direction D between the distal poles Q3 of the small fixed ring 3 and the proximal poles P51 of the first fluctuating ring 51. The distance L12 is the same along the circumferential direction, and is, for example, 0.5 mm.

As shown in Fig. 5, the proximal poles P51 of the first fluctuating ring 51 are written as proximal poles P51 [1], P51 [2]...P51 [7], and distal poles Q51 of the first fluctuating ring 51 are written as distal poles Q51 [1], Q51 [2]... Q51 [7]. A distal envelope curve T51 that connects the plurality of distal poles Q51 of the first fluctuating ring 51 has orthogonal portions T511 and T521 that are orthogonal to the axial direction D, and inclined portions T531 and T541 that are inclined with respect to the axial direction D. The orthogonal portion T511 extends between the distal poles Q51 [4] and Q51 [5]. The orthogonal portion T521 extends between the distal poles Q51 [6], Q51 [7], Q51 [1], Q51 [2], and Q51 [3]. The inclined portion T531 extends between the distal poles Q51 3] and Q51 [4]. The inclined portion T541 extends between the distal poles Q51 [5] and Q51 [6]. The orthogonal portion T511 extends along the portion of the tubular member 2A near the end portion on the greater curvature side of the tubular member 2A in the expansion-contraction direction E. The orthogonal portion T521 extends along the portion of the tubular member 2A from the portion on the greater curvature side of the center of the tubular member 2A in the expansion-contraction direction E to the end portion on the lesser curvature side.

The maximum value of the distance in the axial direction D between the proximal envelope curve S51 and the distal envelope curve T51 will be referred to as a first amplitude G511. The first amplitude G511 corresponds to the distance in the axial direction D between the proximal envelope curve S51 and the orthogonal portion T511 of the distal envelope curve T51. The first amplitude G511 is, for example, 15 mm. The minimum value of the distance in the axial direction D between the proximal envelope curve S51 and the distal envelope curve T51 will be referred to as a second amplitude G521. The second amplitude G521 corresponds to the distance in the axial direction D between the proximal envelope curve S51 and the orthogonal portion T521 of the distal envelope curve T51. The second amplitude G521 is, for example, 10 mm. The first amplitude G511 is larger than the second amplitude G521. The first amplitude G511 and the second amplitude G521 are both larger than the third amplitude G3 (refer to Fig. 2) of the small fixed ring 3.

As shown in Fig. 5, a portion of the first fluctuating ring 51 where the distance between the proximal envelope curve S51 and the distal envelope curve T51 is the first amplitude G511 will be referred to as a first region A511. In particular, the first region A511 includes the proximal pole P51 [4] and the distal poles Q51 [4] and Q51 [5] of the first fluctuating ring 51. A portion of the first fluctuating ring 51 where the distance between the proximal envelope curve S51 and the distal envelope curve T51 is the second amplitude G521 will be referred to as a second region A521. In particular, the second region A521 includes the proximal poles P51 [1], P51 [2], P51 [6], and Q51 [7], and the distal poles Q51 [1] to Q51 [3], Q51 [6], and Q51 [7] of the first fluctuating ring 51.

A center position H51 in the axial direction D between the proximal envelope curve S51 and the distal envelope curve T51 of the first fluctuating ring 51 extends along the circumferential direction while curving with respect to a direction orthogonal to the axial direction D, in accordance with the curving of the distal envelope curve T51 with respect to a direction orthogonal to the axial direction D. As shown in Fig. 2, a minimum distance L30 in the axial direction D between the center position H3 of the small fixed ring 3 and the center position H51 of the first fluctuating ring 51 corresponds to the distance between the positions of the end portions on the lesser curvature side in the expansion-contraction direction E of the center positions H3 and H51. The distance L30 is, for example, 8.75 mm.

### nth fluctuating ring 5n (second fluctuating ring 52 to fifth fluctuating ring 55)

As shown in Fig. 4, the second fluctuating ring 52 is disposed on the distal end 22 (refer to Fig. 1) side with respect to the first fluctuating ring 51. The nth fluctuating ring 5n (except where n = 2) is disposed on the distal end 22 side with respect to the n-1th fluctuating ring 5 (n-1) (expect where n = 2).

As shown in Fig. 6, a proximal envelope curve S5n that connects a plurality of proximal poles P5n of the nth fluctuating ring 5n differs from the first fluctuating ring 51, and curves in the axial direction D. The plurality of proximal poles P5n of the nth fluctuating ring 5n will be written as proximal pole P5n [1], P5n [2]...PSn [7], and distal poles Q5n of the nth fluctuating ring 5n will be written as distal poles Q5n [1], Q5n [2]...Q5n [7]. The proximal envelope curve S5n that connects the plurality of proximal poles P5n of the nth fluctuating ring 5n has an orthogonal portion S51n that is orthogonal to the axial direction D, and inclined portions T53n and T54n that are inclined with respect to the axial direction D. The orthogonal portion S51n extends between the proximal poles P5n [5], P5n [6], P5n [7], P5n [1], P5n [2], and P5n [3]. An inclined portion S53n extends between the proximal poles P5n [3] and P5n [4]. An inclined portion S54n extends between the proximal poles P5n [4] and P5n [5]. A distal envelope curve T5n that connects the plurality of distal poles Q5n of the nth fluctuating ring 5n has orthogonal portions T51n and T52n that are orthogonal to the axial direction D, and inclined portions T53n and T54n that are inclined with respect to the axial direction D. The orthogonal portion T51n extends between the distal poles Q5n [4] and Q5n [5]. The orthogonal portion T52n extends between the distal poles Q5n [6], Q5n [7], Q5n [1], Q5n [2], and Q5n [3]. The inclined portion T53n extends between distal poles Q5n [3] and Q5n [4]. The inclined portion T54n extends between the distal poles Q5n [5] and Q5n [6].

As shown in Fig. 4, the orthogonal portion S51n (S512 and S513) extends along a portion of the tubular member 2A from the portion on the greater curvature side of the center in the expansion-contraction direction E to the end portion on the lesser curvature side. The inclined portions S53n (S532 and S533) and S54n (refer to Fig. 6) extend along a portion of the tubular member 2A near the end portion on the greater curvature side in the expansion-contraction direction E. The orthogonal portion T52n (T522 and T523) extends along a portion of the tubular member 2A from the greater curvature side of the center of the tubular member 2A in the expansion-contraction direction E to the end portion on the lesser curvature side.

As shown in Fig. 6, a maximum value of the distance in the axial direction D between the proximal envelope curve S5n and the distal envelope curve T5n will be referred to as a first amplitude G51n. The first amplitude G51n corresponds to the distance in the axial direction D between the point of intersection of the inclined portions S53n and S54n of the proximal envelope curve S5n, i.e., the proximal pole P5n [4], and the orthogonal portion T51n of the distal envelope curve T5n. The first amplitude G51n is, for example, 15 mm. A minimum value of the distance in the axial direction D between the proximal envelope curve S51 and the distal envelope curve T51 will be referred to as a second amplitude G52n. The second amplitude G52n corresponds to the distance in the axial direction D between the orthogonal portion S51n of the proximal envelope curve S5n and the orthogonal portion T52n of the distal envelope curve T5n. The second amplitude G521 is, for example, 10 mm. The first amplitude G51n is larger than the second amplitude G52n. The first amplitude G51n and the second amplitude G52n are larger than the third amplitude G3 (refer to Fig. 2) of the small fixed ring 3. The first amplitude G51n is the same as the first amplitude G511 of the first fluctuating ring 51. The second amplitude G52n is the same as the second amplitude G521 of the first fluctuating ring 51. Hereinafter, the first amplitudes G511 and G51n will collectively be referred to as the first amplitudes G51. The second amplitudes G521 and G52n will collectively be referred to as the second amplitudes G52. The second amplitudes G52 are larger than the third amplitude G3.

As shown in Fig. 6, a portion of the nth fluctuating ring 5n where the distance between the proximal envelope curve S51 and the distal envelope curve T51 become the first amplitude G51n will be referred to as a first region A51n. In particular, the first region A51n includes the proximal pole P5n [4] and the distal poles Q5n [4] and Q5n [5] of the nth fluctuating ring 5n. A portion of the nth fluctuating ring 5n where the distance between the proximal envelope curve S5n and the distal envelope curve T5n is the second amplitude G52n will be referred to as a second region A52n. In particular, the second region A52n includes the proximal poles P5n [1], P5n [2], P5n [6], and Q5n [7], and the distal poles Q5n [1] to Q5n [3], Q5n [6], and Q5n [7] of the nth fluctuating ring 5n.

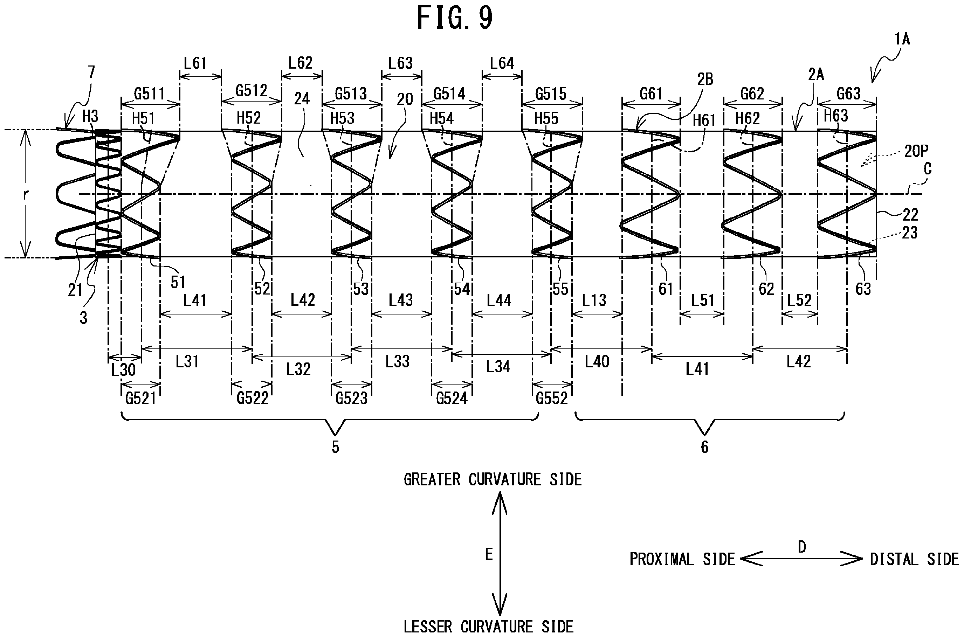

The center position H5n in the axial direction D between the proximal envelope curve S5n and the distal envelope curve T5n of the nth fluctuating ring 5n extends in a direction orthogonal to the axial direction D along the circumferential direction. As shown in Fig. 9, a first distance L31, which is the maximum distance in the axial direction D between the center position H51 of the first fluctuating ring 51 and a center position H52 of the second fluctuating ring 52, corresponds to the distance between the positions of the end portions on the lesser curvature side in the expansion-contraction direction E of the center positions H51 and H52. The first distance L31 is, for example, 21.5 mm. An nth distance L3n, which is the maximum distance in the axial direction D between the center position H5n of the nth fluctuating ring 5n and the center position H5 (n+1) of an n+1th fluctuating ring 5 (n+1), corresponds to the distance between the positions of the end portions on the lesser curvature side in the expansion-contraction direction E of the center positions H5n and H5 (n+1). The nth distance L3n (second distance L32, third distance L33, and fourth distance L34) is the same regardless of the value of n, and is, for example, 19 mm. The first distance L31 is equal to or greater than the nth distance L3n.

As shown in Fig. 4, a maximum distance L41 in the axial direction D between the distal envelope curve T51 of the first fluctuating ring 51 and a proximal envelope curve S52 of the second fluctuating ring 52 corresponds to the distance between the positions of the end portions on the lesser curvature side in the expansion-contraction direction E of the distal envelope curve T51 and the proximal envelope curve S52. The distance L41 is, for example, 11.5 mm. A minimum distance L61 in the axial direction D between the distal envelope curve T51 of the first fluctuating ring 51 and the proximal envelope curve S52 of the second fluctuating ring 52 corresponds to the distance between the positions of the end portions on the greater curvature side in the expansion-contraction direction E of the distal envelope curve T51 and the proximal envelope curve S52. The distance L61 is, for example, 4 mm.

As shown in Fig. 9, a maximum distance L4n (distances L42, L43, and L44) in the axial direction D between the distal envelope curve T5n of the nth fluctuating ring 5n and the proximal envelope curve S5 (n+1) of the n+1th fluctuating ring 5 (n+1) corresponds to the distance between the positions of the end portions on the lesser curvature side in the expansion-contraction direction E of the distal envelope curve T5n and the proximal envelope curve S5 (n+1). The distance L4n is the same regardless of the value of n, and is, for example, 9 mm. The distance L41 is equal to or greater than the distance L4n. A minimum distance L6n (distances L62, L63, and L64) in the axial direction D between the distal envelope curve T5n of the nth fluctuating ring 5n and the proximal envelope curve S5 (n+1) of the n+1th fluctuating ring 5 (n+1) corresponds to the distance between the positions of the end portions on the greater curvature side in the expansion-contraction direction E of the distal envelope curve T5n and the proximal envelope curve S5 (n+1). The distance L6n is the same regardless of the value of n, and is, for example, 4 mm.

The parameters (first distance L31, nth L3n, distances L41, L4n, L61, and L6n, first amplitude G51n, and second amplitude G52n) of the nth fluctuating ring 5n are not limited to the aforementioned embodiment, and may be other values. For example, the distance L61 may be any value greater than 0 mm. The distance L6n may be any value greater than 0 mm. The ratio of the first amplitude G51n to the second amplitude G52n (first amplitude G51n / second amplitude G52n) may be within a range of 1.5 to 2.

### Plurality of large fixed rings 6

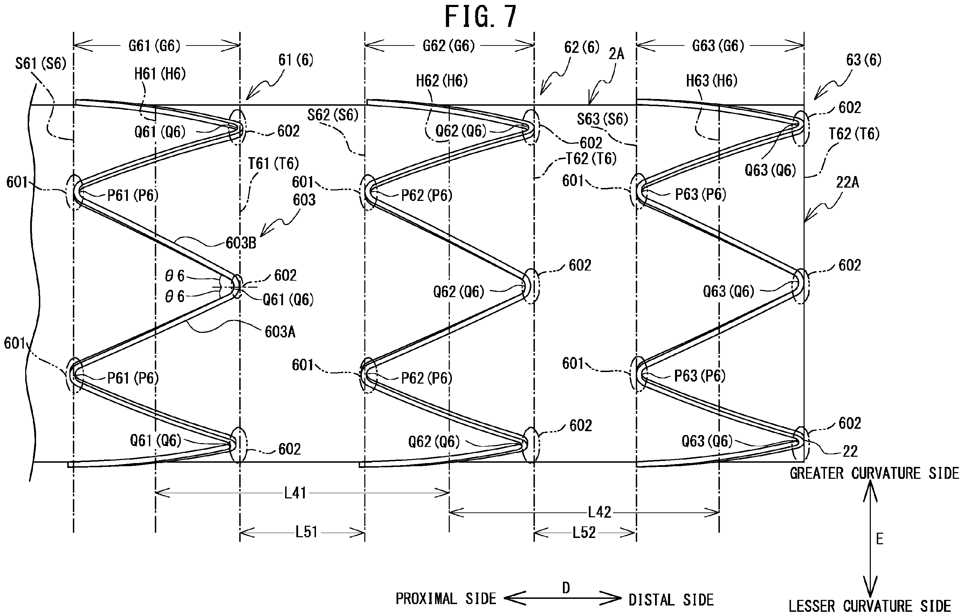

As shown in Fig. 1 and Fig. 7, the plurality of large fixed rings 6 include a first large fixed ring 61, a second large fixed ring 62, and a third large fixed ring 63. The first large fixed ring 61 to the third large fixed ring 63 are arranged in the order of the first large fixed ring 61, the second large fixed ring 62, and the third large fixed ring 63, from the proximal end 21 side toward the distal end 22 side of the tubular member 2A. The shapes of the first large fixed ring 61 to the third large fixed ring 63 are the same. The first large fixed ring 61 is disposed adjacent, on the distal end 22 side, to the fifth fluctuating ring 55. The third large fixed ring 63 is disposed in a position where the positions of the distal end 22 of the tubular member 2A and a plurality of distal poles Q6 of the third large fixed ring 63 are the same in the axial direction D. That is, the plurality of distal poles Q6 of the third large fixed ring 63 are disposed along the distal end 22 of the tubular member 2A.

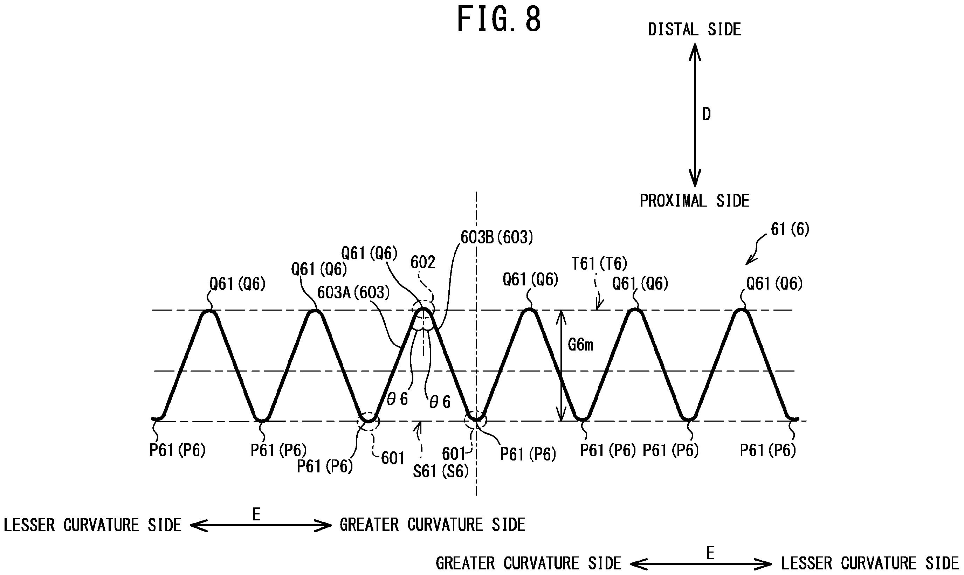

Fig. 8 shows a state in which the first large fixed ring 61 is cut at a portion corresponding to the end portion on the lesser curvature side in the expansion-contraction direction E, and laid out in a plane. As shown in Fig. 7 and Fig. 8, the number of each of a plurality of proximal poles P6 and the plurality of distal poles Q6 of the plurality of large fixed rings 6 is the same for the first large fixed ring 61 to the third large fixed ring 63, and is, for example, 6. A bend cycle W6 is, for example, 1/6. Each of the plurality of large fixed rings 6 has a curved portion 601 near each of the proximal poles P6, a curved portion 602 near each of the plurality of distal poles Q6, and a linear portion 603 extending linearly between the curved portions 601 and 602. A curvature R6 of the curved portions 601 and 602 is the same for the first large fixed ring 61 to the third large fixed ring 63, and is, for example, 0.8. The linear portion 603 extending inclined on the lesser curvature side with respect to the axial direction D from the curved portion 602 toward the proximal side will be referred to as a linear portion 603A. The linear portion 603 extending inclined on the lesser curvature side with respect to the axial direction D from the curved portion 602 toward the proximal side will be referred to as a linear portion 603B. The linear portions 603A and 603B are disposed symmetrically about a reference axis extending in the axial direction D through the curved portion 602 to which they each connect. An angle θ6 formed between the linear portion 603 and the axial direction D is the same for the first large fixed ring 61 to the third large fixed ring 63, and is, for example, 26°.

A proximal envelope curve S6 connecting the plurality of proximal poles P6, and a distal envelope curve T6 connecting a plurality of distal poles Q3 each extend in a direction orthogonal to the axial direction D. The directions in which both the proximal envelope curve S6 and the distal envelope curve T6 extend are parallel to each other. The center position H6 in the axial direction D of the proximal envelope curve S6 and the distal envelope curve T6 is orthogonal to the axial direction D. A fourth amplitude G6, which is the distance between the proximal envelope curve S6 and the distal envelope curve T6, is constant along the entire ring-shaped area of each of the plurality of large fixed rings 6, and is, for example, 15 mm. The fourth amplitude G6 is larger than the third amplitude G3 of the small fixed ring 3, and is the same as the first amplitudes G51 of the plurality of fluctuating rings 5.

A distance L51 in the axial direction D between a distal envelope curve T61 of the first large fixed ring 61 and a proximal envelope curve S62 of the second large fixed ring 62 is the same as a distance L52 in the axial direction D between a distal envelope curve T62 of the second large fixed ring 62 and a proximal envelope curve S63 of the third large fixed ring 63. The distances L51 and L52 are, for example, 4 mm.

As shown in Fig. 9, a maximum distance L40 in the axial direction D between a center position H55 of the fifth fluctuating ring 55 and the center position H61 of the first large fixed ring 61 corresponds to the distance between the positions of the end portions on the lesser curvature side in the expansion-contraction direction E of the center positions H55 and H61. The distance L40 is, for example, 19 mm. As shown in Fig. 7, the distance L41 in the axial direction D between the center position H61 of the first large fixed ring 61 and the center position H62 of the second large fixed ring 62 is the same as a distance L42 in the axial direction D between the center position H62 of the second large fixed ring 62 and the center position H63 of the third large fixed ring 63. The distances L41 and L42 are, for example, 19 mm. The distal end 22 of the tubular member 2A and the distal pole Q63 of the third large fixed ring 63 are disposed in the same position in the axial direction D.

The parameters (bend cycle W6, curvature R6, angle θ6, fourth amplitude G6, and distances L30, L41, L42, L51, and L52) of the plurality of large fixed rings 6 are not limited to the aforementioned embodiment, and may be other values. For example, the bend cycle W6 may be any value within a range of 1/4 to 1/12. The distance L51 may be any value greater than 0 mm. The distance L52 may be any value greater than 0 mm.

### Uncovered section 7

As shown in Fig. 1, the uncovered section 7 is disposed on the proximal end 21 side of the tubular member 2A with respect to the small fixed ring 3. The uncovered section 7 is a strut that extends in a ring shape, and more particularly, is a wire with a rectangular cross-section. The uncovered section 7 extends in a ring shape while alternatingly bending toward the proximal side and toward the distal side in the axial direction D.

As shown in Fig. 2, an end portion on the proximal side in the axial direction D of the uncovered section 7 will be referred to as a tip end 71, and an end portion on the distal side in the axial direction D will be referred to as a base end 72. That is, the uncovered section 7 extends in a ring shape while bending alternatingly toward the tip end 71 side and toward the base end 72 side. The base end 72 is an end portion of the uncovered section 7 on the distal end 22 side of the tubular member 2A, and corresponds to each of a plurality of poles (hereinafter, referred to as a plurality of distal poles Q7) curving in a protruding fashion toward the distal end 22 side. The tip end 71 is an end portion of the uncovered section 7 on the side opposite the distal end 22 with respect to the proximal end 21 of the tubular member 2A, and corresponds to each of a plurality of poles (hereinafter, referred to as a plurality of proximal poles P7) curving in a protruding fashion toward the proximal side in the axial direction D. A proximal envelope curve S7 connecting the plurality of proximal poles P7 and a distal envelope curve T7 connecting the plurality of distal poles Q7 are both orthogonal to the axial direction D. An uncovered section amplitude G7, which is the distance between the proximal envelope curve S7 and the distal envelope curve T7, is constant across the entire ring-shaped area of the uncovered section 7, and is, for example, 12 mm.

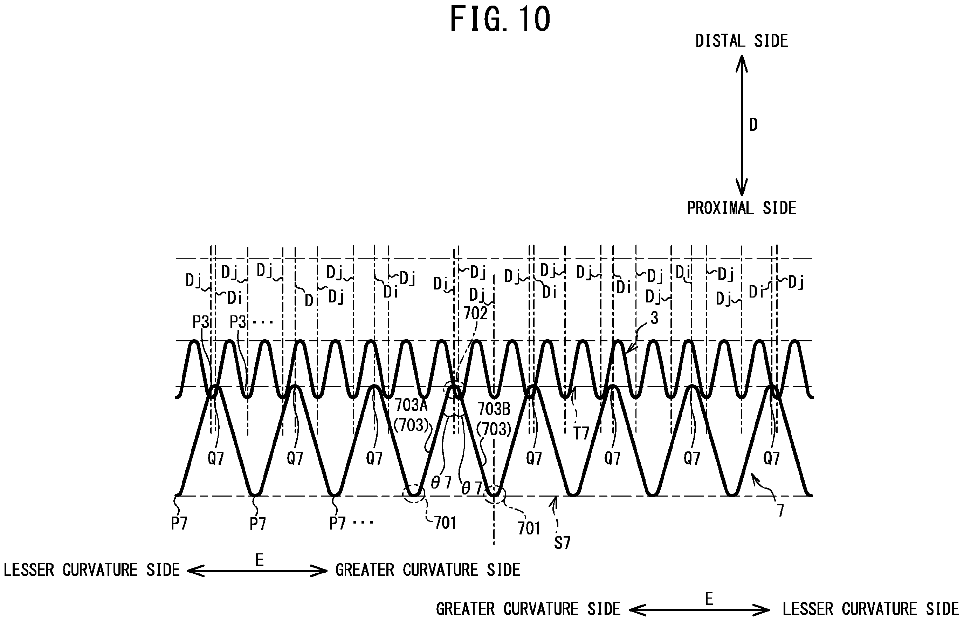

Fig. 10 shows a state in which the uncovered section 7 and the small fixed ring 3 are cut at a portion corresponding to the end portion on the lesser curvature side in the expansion-contraction direction E, and laid out in a plane. The number of each of the plurality of proximal poles P7 and the plurality of distal poles Q7 of the uncovered section 7 is, for example, 8, and a bend cycle W7 is, for example, 1/8. The uncovered section 7 has a curved portion 701 near each proximal pole P7, a curved portion 702 near each distal pole Q7, and a linear portion 703 extending between the curved portions 701 and 702. The linear portion 703 extending inclined on the lesser curvature side with respect to the axial direction D from the curved portion 702 toward the proximal side will be referred to as a linear portion 703A. The linear portion 703 extending inclined on the greater curvature side with respect to the axial direction D from the curved portion 702 toward the proximal side will be referred to as a linear portion 703B. The linear portions 703A and 703B are disposed symmetrically about a reference axis extending in the axial direction D through the curved portion 702 to which they each connect. An angle θ7 formed between the linear portion 703 and the axial direction D is, for example, 12.5°.

As shown in Fig. 2, the uncovered section 7 is such that a portion including the base end 72 is fixed to the inner surface 23 of the proximal end 21 of the tubular member 2A. A portion (including the base end 72) of the uncovered section 7 that is fixed to the tubular member 2A is disposed on the distal end 22 side with respect to the proximal end 21 of the tubular member 2A. A distance L21 in the axial direction D between the proximal end 21 of the tubular member 2A and the base end 72 of the uncovered section 7 is, for example, 4 mm. Therefore, a distance L11 in the axial direction D between the base end 72 of the uncovered section 7 and the plurality of proximal poles P3 of the small fixed ring 3 is also 4 mm.

The positions in the circumferential direction of the distal poles Q7 of the uncovered section 7 and the plurality of proximal poles P3 of the small fixed ring 3 are different. More specifically, as shown in Fig. 10, a virtual straight line Di extending in the axial direction D through the distal poles Q7 of the uncovered section 7, and a virtual straight line Dj extending in the axial direction D through the plurality of proximal poles P3 of the small fixed ring 3 are not disposed on a straight line.

As shown in Fig. 2, a portion excluding a portion (including the base end 72) of the uncovered section 7 that is fixed to the base end 72 is disposed on the proximal end side in the axial direction D with respect to the proximal end 21 of the tubular member 2A, in other words, on the side opposite the distal end 22 with respect to the proximal end 21 of the tubular member 2A. Hereinafter, this portion will be referred to as a protruding portion 70. A distance L20 in the axial direction D of the protruding portion 70 corresponds to the distance in the axial direction D between the tip end 71 of the uncovered section 7 and the proximal end 21 of the tubular member 2A. The distance L20 is, for example, 8 mm.

In the protruding portion 70, a direction from the base end 72 toward the tip end 71 will be referred to as the extending direction F. The extending direction is inclined with respect to the axial direction D. More specifically, the extending direction F extends inclined in a direction away from the center line C. Therefore, the diameter of the proximal envelope curve S7 connecting the plurality of proximal poles P7 of the tip end 71 becomes larger than the diameter of the distal envelope curve T7 connecting the plurality of distal poles Q7 of the base end 72.

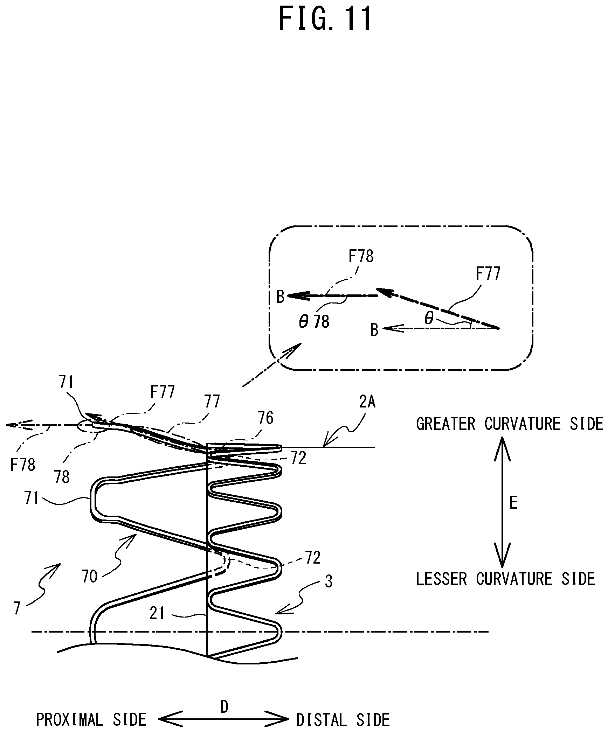

Further details are as follows. As shown in Fig. 11, the uncovered section 7 is curved. The uncovered section 7 has a base end portion 76, a first portion 77, and a second portion 78. The base end portion 76 is a portion fixed to the tubular member 2A. The first portion 77 is connected to the tip end of the base end portion 76 and extends along an extending direction F77 toward the proximal side. The second portion 78 is connected to the tip end of the first portion 77 and extends along an extending direction F78 toward the proximal side. The tip end of the second portion 78 corresponds to the tip end 71 of the uncovered section 7. The angles formed between each of the extending directions F77 and F78 and a reference direction B parallel to the center line C are written as a first angle Θ77 and a second angle Θ78. The first angle Θ77 is, for example, 12°. The second angle Θ78 is, for example, 0°. The second angle Θ78 is smaller than the first angle θ77.

The parameters (the uncovered section amplitude G7, the bend cycle W7, the curvature R7, the angle θ7, the distances L11, L20, and L21, the first angle θ77, and the second angle θ78) of the uncovered section 7 are not limited to the aforementioned embodiment, and may be other values. For example, the bend cycle W7 may be any value within a range of 1/4 to 1/12. The distance L11 may be any value within a range of 0 mm to 4 mm. The distance L21 may be any value within a range of 0 mm to 4 mm.

### Method of use

The stent graft 1A is fitted folded up in a delivery system. The delivery system is inserted into a body through an incision in a blood vessel in the base of a leg. At this time, the proximal side of the stent graft 1A, in other words, the side where the uncovered section 7 is provided in the axial direction D, faces the direction of travel of the delivery system. After the stent graft 1Ais guided to the affected area, it is then pushed out of the delivery system and placed inside the blood vessel. The tubular member 2A of the stent graft 1A then expands due to the elastic force of the plurality of stent rings 2B.

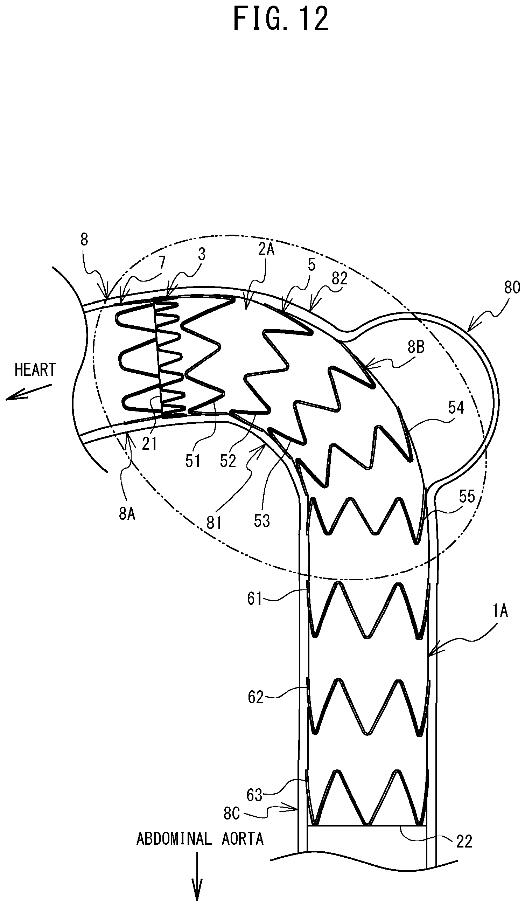

As shown in Fig. 12, the stent graft 1A closely contacts the inside of the thoracic aorta 8 and curves along an aortic arch 8B. The uncovered section 7 of the stent graft 1A closely contacts the position of the thoracic aorta that is closer to the heart, in the aortic arch 8B. The lesser curvature side in the expansion-contraction direction E of the tubular member 2A of the stent graft 1A is disposed on a lesser curvature portion 81 side of the aortic arch 8B. An end portion on the lesser curvature side of each of the plurality of fluctuating rings 5 closely contacts the lesser curvature portion 81 that becomes the sharply curved portion of the aortic arch 8B. The greater curvature side in the expansion-contraction direction E of the tubular member 2A is disposed on a greater curvature portion 82 side of the aortic arch 8B. An end portion on the greater curvature side of each of the plurality of fluctuating rings 5 closely contacts the greater curvature portion 82 of the aortic arch 8B. The plurality of large fixed rings 6 closely contact the position of a descending aorta 8C closer to the abdominal aorta, of the thoracic aorta 8.

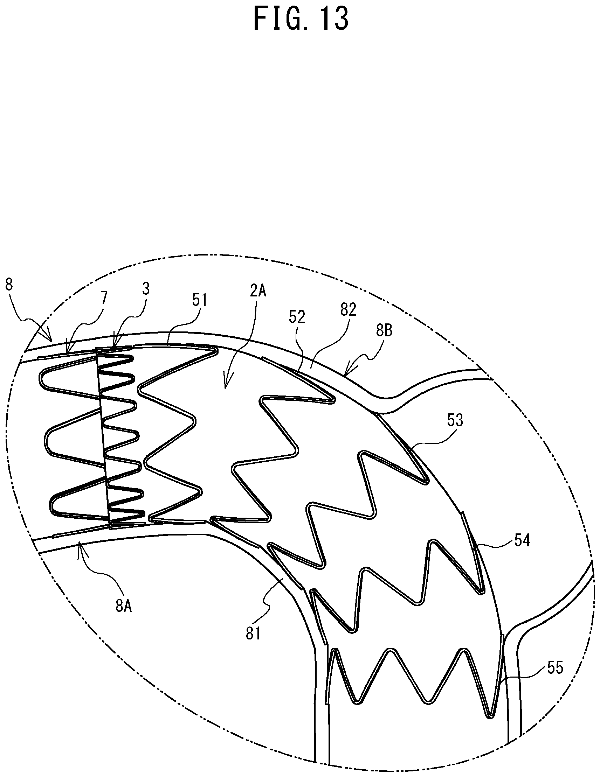

As shown in Fig. 13, the tubular member 2A curves at the lesser curvature portion 81 that becomes the sharply curved portion of the aortic arch 8B, by contracting the portions between the plurality of fluctuating rings 5, of the portions on the lesser curvature side in the expansion-contraction direction E. Here, as shown in Fig. 9, the distance L41 on the lesser curvature side between the first fluctuating ring 51 and the second fluctuating ring 52 is greater than the distance L61 on the greater curvature side. The distance L42 on the lesser curvature side between the second fluctuating ring 52 and the third fluctuating ring 53 is greater than the distance L62 on the greater curvature side. The distance L43 on the lesser curvature side between the third fluctuating ring 53 and the fourth fluctuating ring 54 is greater than the distance L63 on the greater curvature side. The distance L44 on the lesser curvature side between the fourth fluctuating ring 54 and the fifth fluctuating ring 55 is greater than the distance L64 on the greater curvature side. Therefore, the tubular member 2A contracts easier and curves easier on the lesser curvature side than on the greater curvature side at the position where the plurality of fluctuating rings 5 are fixed. Therefore, with the stent graft 1A, the tubular member 2A can be made to closely contact the lesser curvature portion 81 that becomes the sharply curved portion of the aortic arch 8B.

As shown in Fig. 12, the stent graft 1A protects the thoracic aorta 8 by inhibiting, with the tubular member 2A, the flow of blood to the portion of an aortic aneurysm 80 formed in the aortic arch 8B. By combining the uncovered section 7 of the stent graft 1A with a plurality of stents, the opening 21A formed in the proximal end 21 of the tubular member 2A can be made to more suitably closely contact the inner wall of the blood vessel. Therefore, the stent graft 1A can inhibit the occurrence of a phenomenon in which blood leaks out of the opening of the proximal end 21 of the tubular member 2A and flows into the aortic aneurysm 80, i.e., an end leak.

### Operations and effects of the present embodiment

The stent graft 1A can cause the portion of the tubular member 2A where the plurality of fluctuating rings 5 are fixed to closely contact the inner wall of the lesser curvature portion 81 that is the sharply curved portion of the aortic arch 8B. Also, the stent graft 1A can cause the uncovered section 7 to closely contact the inner wall of an ascending aorta 8A of the aortic arch 8B. Therefore, with the stent graft 1A, the opening 21A formed in the proximal end 21 of the tubular member 2A can be made to suitably closely contact the inner wall of the blood vessel, so the stent graft 1A can inhibit an end leak from occurring.

The small fixed ring 3 is adjacent on the distal end 22 side of the tubular member 2A with respect to the uncovered section 7. The plurality of fluctuating rings 5 are adjacent on the distal end 22 side of the tubular member 2Awith respect to the small fixed ring 3. That is, the small fixed ring 3 is disposed between the uncovered section 7 and the plurality of fluctuating rings 5 provided to suitably bend the tubular member 2A. The third amplitude G3 of the small fixed ring 3 is smaller than the first amplitudes G511 and G51n of the plurality of fluctuating rings 5. Also, the bend cycle W3 of the small fixed ring 3 is larger than the bend cycle W5 of the plurality of fluctuating rings 5. Therefore, the small fixed ring 3 can bring the opening 21A of the proximal end 21 of the tubular member 2A into close contact with the blood vessel with greater elastic force. Therefore, with the stent graft 1A, the opening 21A formed in the proximal end 21 of the tubular member 2A can be made to more suitably closely contact the inner wall of the blood vessel by the uncovered section 7 and the small fixed ring 3.

The position in the circumferential direction of the plurality of distal poles Q7 corresponding to the base end 72 of the uncovered section 7 and the position in the circumferential direction of the plurality of proximal poles P3 of the small fixed ring 3 are different. As a result, the stent graft 1A can inhibit contact between the uncovered section 7 and the small fixed ring 3 even if the plurality of distal poles Q7 of the uncovered section 7 and the plurality of proximal poles P3 of the small fixed ring 3 are close to each other.

The uncovered section 7 is such that the extending direction F from the base end 72 toward the tip end 71 extends in a direction away from the center line C of the tubular member 2A. In this case, when the stent graft 1A is disposed inside the blood vessel, the uncovered section 7 more easily contacts the inner wall of the blood vessel. Therefore, with the stent graft 1A, the uncovered section 7 can be made to closely contact the inner wall of the blood vessel by the elastic force generated in the uncovered section 7. Also, the uncovered section 7 can reduce the possibility of the opening of the proximal end 21 of the tubular member 2A becoming misaligned with respect to the blood vessel.

The uncovered section 7 has the first portion 77 in which the angle of the extending direction F with respect to the reference direction B is the first angle Θ77, and the second portion 78 in which the angle of the extending direction F with respect to the reference direction B is the second angle Θ78 that is smaller than the first angle Θ77. In this case, when the stent graft 1A is disposed inside the blood vessel, the first portion 77 to the second portion 78 contact the inner wall of the blood vessel more easily than does the tip end 71 of the uncovered section 7. That is, by contacting the blood vessel with the whole of the first portion 77 to the second portion 78, the uncovered section 7 can reduce the possibility of only the tip end 71 contacting the blood vessel. Therefore, the stent graft 1A can reduce the possibility of the tip end 71 of the uncovered section 7 damaging the inner wall of the blood vessel.

The stent graft 1A is provided with the plurality of large fixed rings 6 having the fourth amplitude G6. The plurality of large fixed rings 6 are disposed on the distal end 22 side with respect to the plurality of fluctuating rings 5 and the small fixed ring 3. In this case, the stent graft 1A causes the plurality of large fixed rings 6 to closely contact a portion (for example, the descending aorta 8C) extending linearly from the sharply curved portion of the blood vessel. The fourth amplitude G6 of the plurality of large fixed rings 6 is larger than the third amplitude G3 of the small fixed ring 3. Therefore, the plurality of large fixed rings 6 can press the tubular member 2A with an appropriate amount of force to bring the tubular member 2A into close contact with the linear extending descending aorta 8C.

The third amplitude G3 of the small fixed ring 3 is equal to or smaller than the second amplitudes G521 and 52n of the plurality of fluctuating rings 5. In this case, the force that expands the tubular member 2A outward can be increased by the elastic force of the small fixed ring 3. Therefore, the stent graft 1A can further increase the force that brings the opening 21A of the proximal end 21 of the tubular member 2A into close contact with the inner wall of the blood vessel.

The base end 72 of the uncovered section 7 is fixed with the sutures or the like to the inner surface 23 of the proximal end 21 of the tubular member 2A. In this case, elastic force of the uncovered section 7 expands the opening 21A of the proximal end 21 of the tubular member 2A outward. Therefore, the stent graft 1A can suitably bring the proximal end 21 of the tubular member 2A into close contact with the inner wall of the blood vessel. Also, the plurality of stent rings 2B are fixed to the outer surface 24 of the tubular member 2A. Therefore, the stent graft 1A can reduce the possibility that various devices that pass through the inside of the tubular member 2A will catch on the plurality of stent rings 2B.

The plurality of stent rings include the five fluctuating rings 5 and the one small fixed ring 3. In this case, the stent graft 1A can further increase the closeness of contact of the tubular member 2A with respect to the inner wall of the lesser curvature portion 81 that is the sharply curved portion of the aortic arch 8B by the five fluctuating rings 5. Also, with the stent graft 1A, the opening 21A of the proximal end 21 of the tubular member 2A can be brought into suitably close contact with the blood vessel by the one small fixed ring 3.

The proximal envelope curve S51 connecting the plurality of proximal poles P51 of the first fluctuating ring 51 extends in the circumferential direction and is orthogonal to the axial direction D. That is, the plurality of proximal poles P51 of the first fluctuating ring 51 are arranged on a virtual line (the proximal envelope curve S51) that extends in the circumferential direction along the outer surface 24 of the tubular member 2A and is orthogonal to the axial direction D. In this case, the small fixed ring 3 and the first fluctuating ring 51 are arranged maintaining a constant distance from each other, so the force that expands the tubular member 2A outward is particularly strong at the proximal end 21 of the tubular member 2A. Therefore, the stent graft 1A can further increase the force that brings the opening 21A of the proximal end 21 of the tubular member 2A into close contact with the inner wall of the blood vessel.

Note that each amplitude G of the plurality of stent rings 2B is defined as the distance in the axial direction D between the proximal envelope curve S connecting the plurality of proximal poles P on the proximal end 21 side, and the distal envelope curve T connecting the plurality of distal poles Q on the distal end 22 side. As a result, the shapes of the plurality of stent rings 2B that enable close contact with the inner wall of the blood vessel can easily be defined on the basis of the amplitudes G. Note that with the plurality of fluctuating rings 5, the first amplitude G51n on the greater curvature side is greater than the second amplitude G52n on the lesser curvature side. As a result, with the stent graft 1A, the portion of the tubular member 2A to which the plurality of fluctuating rings 5 are fixed can be made to closely contact the inner wall of the lesser curvature portion 81 that is the sharply curved portion of the aortic arch 8B.

### Modified examples

The present invention is not limited to the aforementioned embodiment; various modifications are possible. The stent graft 1A is not limited to being used placed in the aortic arch, and may be used placed in another portion of a blood vessel.

The amplitudes of the plurality of fluctuating rings 5 may gradually become smaller from the largest first amplitude G51 toward the smallest second amplitude G52. In this case, the proximal envelope curve G5 and the distal envelope curve T5 may extend linearly in a direction toward one another.

The shape of the uncovered section 7 is not limited to that in the aforementioned embodiment, and may be another shape. For example, the tip end 71 of the uncovered section 7 may be bent in a rectangular shape. The base end 72 of the uncovered section 7 may be fixed to the proximal end 21 of the tubular member 2A. The method for fixing the uncovered section 7 to the tubular member 2A is not limited to that in the aforementioned embodiment. For example, the uncovered section 7 may be fixed to the tubular member 2A by adhesive or the like.

The number and sequence of the uncovered section 7, the small fixed ring 3, the plurality of fluctuating rings 5, and the plurality of large fixed rings 6 are not limited to those in the aforementioned embodiment. For example, they may be arranged in the order of the uncovered section 7, the first fluctuating ring 51, the small fixed ring 3, the nth fluctuating ring 5n, and the plurality of large fixed rings 6. The small fixed ring 3 may be provided between the plurality of fluctuating rings 5 and the plurality of large fixed rings 6. The small fixed ring 3 may be provided on the distal end 22 side of the tubular member 2A with respect to the third large fixed ring 63. The small fixed ring 3 may be provided in plurality. An uncovered section may also be provided on the distal end 22 of the tubular member 2A.

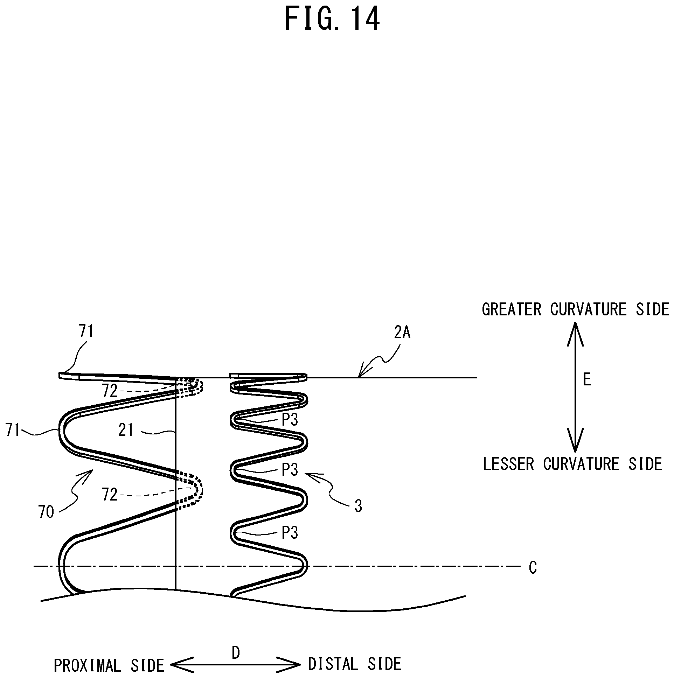

As shown in Fig. 14, the base end 72 of the uncovered section 7 need not be positioned at the end portion of the small fixed ring 3 that is on the proximal end 21 side of the tubular member 2A, i.e., on the distal end 22 side with respect to the plurality of proximal poles P3. That is, the base end 72 of the uncovered section 7 and the plurality of proximal poles P3 of the small fixed ring 3 need not overlap in the axial direction D. Note that the tip end 71 of the uncovered section 7 is positioned on the side opposite the distal end 22 side with respect to the plurality of proximal poles P3 of the small fixed ring 3.

The positions in the circumferential direction of the plurality of distal poles Q7 of the uncovered section 7 and the plurality of proximal poles P3 of the small fixed ring 3 may be the same. More specifically, the virtual straight line Di extending in the axial direction D through each of the distal poles Q7 of the uncovered section 7, and the virtual straight line Dj extending in the axial direction D through each of the plurality of proximal poles P3 of the small fixed ring 3 may be arranged in a straight line. The plurality of distal poles Q7 of the uncovered section 7 and the plurality of proximal poles P3 of the small fixed ring 3 may contact each other.

The uncovered section 7 may have a plurality of portions at different sites with different angles. The uncovered section 7 may extend linearly from the base end 72 toward the tip end 71. Alternatively, the uncovered section 7 may extend parallel to the axial direction D. In this case, the uncovered section 7 need not be inclined with respect to the axial direction D.

The plurality of stent rings 2B may include only the small fixed ring 3 and the plurality of fluctuating rings 5, and not include the plurality of large fixed rings 6. In this case, the plurality of fluctuating rings 5 may be disposed up to near the distal end 22 of the tubular member 2A.

The uncovered section 7 may be such that the base end 72 is fixed to the outer surface 24 of the proximal end 21 of the tubular member 2A. In this case, the uncovered section 7 can easily be attached to the tubular member 2A. The plurality of stent rings 2B may be fixed to the inner surface 23 of the tubular member 2A. In this case, the tubular member 2A can be more suitably expanded outward by the plurality of stent rings 2B, thereby enabling the tubular member 2A to closely contact the blood vessel.

The first amplitudes G511 and G51n of the plurality of fluctuating rings 5 may be different from the fourth amplitude G6 of the plurality of large fixed rings 6. The third amplitude G3 of the small fixed ring 3 may be larger than the second amplitudes G521 and 52n of the plurality of fluctuating rings 5, or the third amplitude G3 and the second amplitudes G521 and 52n may be the same.

The number of the plurality of fluctuating rings 5 and the plurality of large fixed rings 6 is not limited to those in the aforementioned embodiment. Therefore, the number of the plurality of fluctuating rings 5 may be any number from 2 to 4 or 6 or more. Also, the plurality of stent rings 2B may include only the first fluctuating ring 51 or the second fluctuating ring 52. That is, the stent graft 1A may have only one fluctuating ring 5.

The positions in the axial direction D of the plurality of proximal poles P51 of the first fluctuating ring 51 need not be the same. More specifically, having at least a portion of the proximal envelope curve S51 that connects the plurality of proximal poles P51 of the first fluctuating ring 51 be inclined with respect to a direction orthogonal to the axial direction D is sufficient. For example, the first fluctuating ring 51 may have the same shape as the nth fluctuating ring 5n.

The plurality of proximal poles P3 of the small fixed ring 3 may be disposed on the distal side with respect to the proximal end 21 of the tubular member 2A. That is, the plurality of proximal poles P3 of the small fixed ring 3 and the proximal end 21 of the tubular member 2A may be disposed in different positions in the axial direction D. The plurality of distal poles Q6 of the third large fixed ring 63 disposed on the distal-most end may be disposed on the proximal side with respect to the distal end 22 of the tubular member 2A. That is, the plurality of distal poles Q6 of the third large fixed ring 63 and the distal end 22 of the tubular member 2A may be disposed at different positions in the axial direction D.

The curvature of the curved portion 701 and the curvature of the curved portion 702 of the uncovered section 7 may be the same or they may be different. The curvature of the curved portion 701 may be the same for all of the plurality of proximal poles P7, or it may be different for each of the plurality of proximal poles P7.

The positions in the circumferential direction of the distal poles Q7 of the uncovered section 7 and the plurality of proximal poles P3 of the small fixed ring 3 may be the same. In this case, with the stent graft 1A, the overlapping portion of the uncovered section 7 and the small fixed ring 3 allows the tubular member 2A to more efficiently expand outward and closely contact the blood vessel.

The plurality of proximal poles P3 of the small fixed ring 3 need not be disposed in the same position in the axial direction D. Having at least a portion of the proximal envelope curve S3 that connects the plurality of proximal poles P3 be inclined with respect to the axial direction D is sufficient. Similarly, the plurality of distal poles Q3 of the small fixed ring 3 need not be disposed at the same position in the axial direction D. Having at least a portion of the distal envelope curve T3 that connects the plurality of distal poles Q3 be inclined with respect to the axial direction D is sufficient. The third amplitude G3 that is the distance between the proximal envelope curve S3 and the distal envelope curve T3 may fluctuate in accordance with the position in the circumferential direction of the small fixed ring 3.

## Claims

1. A stent graft comprising:
a tubular member extending between a proximal end that is an end portion on one side in a predetermined axial direction, and a distal end that is an end portion on the other side in the axial direction;
a plurality of stent rings fixed to a side wall of the tubular member and formed of struts extending in a ring shape in a circumferential direction of the tubular member, while alternatingly bending toward the proximal end side and toward the distal end side, the plurality of stent rings including at least
a fluctuating ring including a first region in which an amplitude in the axial direction is a first value, and a second region in which an amplitude is a second value that is smaller than the first value, and
a small fixed ring in which a maximum value of an amplitude is a third value that is smaller than the first value; and
an uncovered section formed of a strut, at least a portion of which is disposed on a side opposite the distal end side with respect to the proximal end, the strut extending in a ring shape while alternatingly bending toward a base end side that is an end portion on the distal end side, and toward a tip end side that is an end portion on the side opposite the distal end side.

2. The stent graft according to claim 1, wherein:
the small fixed ring is adjacent on the distal end side with respect to the uncovered section; and
the fluctuating ring is adjacent on the distal end side with respect to the small fixed ring.

3. The stent graft according to claim 1, wherein:
the base end of the uncovered section is positioned on the distal end side with respect to the end portion on the proximal end side of the small fixed ring, and the tip end of the uncovered section is positioned on the side opposite the distal end side with respect to the end portion on the proximal end side of the small fixed ring; and
the fluctuating ring is adjacent on the distal end side with respect to the small fixed ring.

4. The stent graft according to claim 2 or 3, wherein:
positions in the circumferential direction of a plurality of poles of the base end of the uncovered section and a plurality of poles on the proximal end side of the small fixed ring are different.

5. The stent graft according to claim 2 or 3, wherein:
positions in the circumferential direction of a plurality of poles of the base end of the uncovered section and a plurality of poles on the proximal end side of the small fixed ring are the same.

6. The stent graft according to any one of claims 1 to 5, wherein:
the uncovered section is such that an extending direction from the base end toward the tip end extends in a direction away from a center line passing through a center of the tubular member and extending in the axial direction.

7. The stent graft according to claim 6, wherein:
the uncovered section includes
a first portion where an angle of the extending direction with respect to a reference direction is a first angle, the reference direction extending on the side opposite the distal end side with respect to the proximal end and being parallel to the center line; and
a second portion including the tip end and where an angle of the extending direction with respect to the reference direction is smaller than the first angle.

8. The stent graft according to any one of claims 1 to 7, wherein:
the plurality of stent rings further include a large fixed ring in which a maximum value of an amplitude is a fourth value that is larger than the third value, and which is disposed on the distal end side of the fluctuating ring and the small fixed ring.

9. The stent graft according to any one of claims 1 to 8, wherein:
the uncovered section is such that the base end is fixed to an inner surface of the proximal end of the tubular member.

10. The stent graft according to any one of claims 1 to 9, wherein:
the plurality of stent rings are fixed to an outer surface of the tubular member.

11. The stent graft according to any one of claims 1 to 10, wherein:
the plurality of stent rings are fixed to an inner surface of the tubular member.

12. The stent graft according to any one of claims 1 to 11, wherein:
the third value is smaller than the second value.

13. The stent graft according to any one of claims 1 to 12, wherein:
there are a plurality of the fluctuating rings and the single small fixed ring.

14. The stent graft according to any one of claims 1 to 13, wherein:
the plurality of poles on the proximal end side of the fluctuating ring are arranged on a virtual line extending in a direction orthogonal to the axial direction along the circumferential direction.
